# FASCICULE DE BREVET EUROPEEN

(11) **EP 0 264 303 B1**
(45) Date de publication et mention de la délivrance du brevet: **15.06.1994**
(21) Numéro de dépôt: 87401711.4
(22) Date de dépôt: 23.07.1987
(51) Int. Cl.: A61F 5/01, A61H 1/00

(54) **Attelle orthopédique**
Orthopädische Schiene
Orthopaedic splint

(30) Priorité: 14.08.1986 FR 8611771
(43) Date de publication de la demande: 20.04.1988
(73) Titulaire: Dumoutier, Gérard Michel, F-74700 Sallanches (FR)
(72) Inventeur: Dumoutier, Gérard Michel, F-74700 Sallanches (FR)
(74) Mandataire: Ballot, Paul Denis Jacques

(56) Documents cités:
- EP-A- 0 192 181
- DE-A- 3 030 712
- FR-A- 1 519 668
- FR-A- 2 305 166
- FR-A- 2 552 660
- GB-A- 505 775
- US-A- 3 086 522
- US-A- 4 237 873
- US-A- 4 256 097

## Description

La présente invention concerne les attelles orthopédiques destinées a participer au traitement de tous les troubles pathologiques des articulations, qu'ils soient d'origine traumatique, rhumatologique, neurologique, virale ou microbienne.

On sait qu'un très grand nombre de personnes sont atteintes de troubles articulaires tels que : entorses, fractures, arthroses, arthrites, hémiplégies, mal de Pott, ou autres.

A un moment donné de leur traitement, tous ces sujets relèvent d'immobilisations, soit dans un but curatif, comme par exemple dans les entorses, les fractures et les arthroses, soit dans un but préventif des déformations articulaires, comme par exemple dans les polyarthrites rhumatoïdes ou le mal de Pott, soit encore dans un but préventif des attitudes vicieuses liées aux contractures musculaires d'origine neurologique, comme par exemple dans les hémiplégies.

Le but de telles immobilisations est de traiter le symptôme et, parallèlement, mais sauf parfois pour les troubles neurologiques, de supprimer la douleur.

Le traitement est réalisé en deux temps : une phase d'immobilisation réalisée avec des moyens de contention divers suivie, après ablation de ces derniers, d'une phase de récupération fonctionnelle, c'est-à-dire, dans la plupart des cas, de kinésithérapie.

L'immobilisation concerne toujours un ou plusieurs segments de membre et au moins une articulation.

Si l'immobilisation parvient à traiter le symptôme et à supprimer la douleur, elle porte en elle-même sa propre contradiction, à savoir ce que l'on appelle "enraidissement articulaire".

En effet, dans l'état actuel de la technique, tous les moyens de contention utilisés pour réaliser une immobilisation sont statiques et ne permettent donc aucun mouvement des articulations concernées.

Or, on sait que l'immobilisation d'un segment de membre et d'une articulation entraîne un raidissement articulaire consécutif à l'absence de mouvement et, surtout, de stimulation neuromotrice. Pendant la phase de récupération fonctionnelle, cet état de fait entraîne des douleurs importantes des systèmes musculo-tendineux et capsulo-ligamentaires.

Ces douleurs rendent complexe, longue et difficile la récupération des amplitudes articulaires physiologiques. On se trouve devant une contradiction selon laquelle l'immobilisation entraîne la disparition de la douleur, mais entraîne aussi un raidissement articulaire, et donc la réapparition de la douleur, ce qui limite la récupération fonctionnelle.

Dans l'état actuel de la technique, les moyens de contention utilisés pour réaliser une immobilisation sont rigides, semi-souples ou souples.

Les moyens de contention rigides sont le plâtre et les matières thermoformables. Les plâtres, qu'ils se présentent sous la forme de gouttière, de bivalve ou de plâtre fermé, englobent toujours au moins une articulation. Pour leur part, les attelles en matière thermoformable peuvent se présenter, soit en une seule partie, soit en deux parties reliées par une tige de fer articulée.

Quand aux attelles semi-souples, elles se présentent sous la forme d'un tissu élastique renforcé par des baleines métalliques.

Ces deux premiers types de contention rigides et semi-souples présentent les inconvénients qui ont été décrits ci-dessus, du fait qu'ils ne permettent aucun mouvement articulaire. Même si les attelles thermoformables en deux parties sont articulées, ceci n'a pour objectif que de fixer l'articulation dans la position d'immobilisation choisie, et encore d'une manière restrictive, puisque de telles attelles n'utilisent la plupart du temps pour leurs déplacements qu'un seul plan de l'espace, au mieux deux, mais jamais trois.

On connaît encore des contentions souples réalisées en une matière appelée "élastoplast", et il est vrai que, dans ce cas de figure, l'articulation garde une possibilité de mobilité, mais ce moyen de contention n'est utilisé que dans des cas mineurs et, en outre, les risques d'allergie et les troubles trophiques sont fréquents.

Dans tous les cas, aucun de ces moyens de contention n'a la possibilité intrinsèque de réaliser des stimuli au niveau des articulations.

Enfin, on connait des attelles comprenant deux organes de contention dont chacun peut être attaché à une partie du corps du patient et qui sont reliés entre eux par une pièce mécanique, pièce dont la longueur totale au repos est réglable et qui comprend deux tiges déplaçables axialement l'une par rapport à l'autre. Une telle attelle est décrite dans la demande de brevet européen EP-A-0.192.181.

S'il est exact qu'un tel dispositif permet au patient de faire travailler l'articulation concernée, il ne résoud pas l'ensemble des problèmes exposés ci-avant et, en particulier, il ne permet pas de régler à volonté et de manière précise les mouvements qui sont exécutés. En outre, les contractions musculaires qu'il suppose de la part du patient sont totalement exclues dans un grand nombre de cas.

Par ailleurs, le document AU-A-19460/70 décrit des attelles comprenant notamment deux tiges déplaçables axialement l'une par rapport à l'autre et des moyens actifs pour imprimer auxdites tiges, des mouvements, tendant à modifier leur position axiale relative.

Aussi un but de la présente invention est d'éviter le raidissement articulaire en recréant artificiellement des micro-mouvements physiologiques d'étirement/relâchement (ou de pression/depression) des articulations, que l'on appelera par la suite des "pompages", qui interviennent sur les systèmes musculo-tendineux et capsulo-ligamentaire et qui visent à remplacer le "balayage" articulaire responsable de la nutrition du cartilage.

Les pompages du système musculo-tendineux permettent de conserver, par phénomène réflexe, une élasticité satisfaisante des muscles et des tendons. Pour leur part, les pompages du système capsulo-ligamentaire permettent de conserver, également par phénomène réflexe, la sécrétion du liquide synovial qui est à la fois lubrifiant des surfaces articulaires en présence et nourissier du cartilage articulaire.

Un autre but de la présente invention est de pouvoir régler à volonté tous les paramètres d'un pompage physiologique, à savoir son rythme, son amplitude, sa direction et sa force, ces paramètres étant variables en fonction de l'atteinte, de l'articulation touchée et de la morphologie du sujet.

Selon l'invention, ces buts sont atteints grâce à une attelle orthopédique, du genre comprenant deux organes de contention dont chacun peut être attaché à une partie du corps du patient et qui sont reliés entre eux par une pièce mécanique, pièce dont la longueur totale est réglable et qui comprend deux tiges déplaçables axialement l'une par rapport à l'autre, comprenant des moyens actifs (5, 6, 8) pour imprimer à ces tiges (1, 9) indépendamment de la volonté du patient, des impulsions de pompage tendant à modifier leur position axiale relative, cette attelle étant caractérisé par le fait que l'extrémité libre de chacune desdites tiges (1, 9) est munie d'une rotule (2, 10) qui peut tourner dans une cuvette sphérique (3, 11) solidaire de l'organe de contention (A, A') correspondant et qui peut être bloquée dans une position choisie à l'avance au moyen de vis de blocage (4, 12).

On comprend que cette disposition, à elle seule, permet de réaliser des pompages alternatifs suivant une direction donnée, déterminée par le symptôme, afin d'entretenir un état trophique satisfaisant de tous les systèmes concernés et, en conséquence, d'éviter l'enraidissement articulaire et de conserver une articulation souple, ce qui conduit à une phase de récupération fonctionnelle rapide et indolore, cependant que le réglage de la longueur de la pièce mécanique reliant les organes de contention permet d'adapter aisément le dispositif en fonction des problèmes posés.

Avantageusement, les moyens utilisés pour imprimer aux tiges spécifiées ci-avant des impulsions de "pompage" comprennent un cylindre axial dans lequel coulisse un piston, ce dernier et son cylindre étant chacun solidaire de l'une des tiges en question, ainsi qu'une tubulure d'admission périodique de fluide sous pression dans le fond du cylindre.

Il est clair que l'introduction périodique de fluide sous pression entre le fond du cylindre et le piston repousse ce dernier à intervalles réguliers et sur une longueur que l'on peut choisir à volonté en réglant la pression du fluide, ce qui produit l'effet de "pompage" recherché. Il y a lieu de noter à ce sujet que, dans la plupart des cas, il est inutile de réaliser une contre-pression de fluide pour faire revenir le piston à sa position initiale. En effet, la pression pneumatique met en tension le système ligamentaire, et c'est ce dernier qui se charge de repousser le piston lorsque l'on annule la pression du fluide. En revanche, si le système est trop "laxe", on peut envisager un ressort d'assistance pour ramener le piston à sa position initiale. Il faut remarquer également que l'on peut, bien évidemment, choisir le rythme, la direction et la force du "pompage", cette dernière ne devant d'ailleurs pas excéder 4 kg environ pour des raisons anatomo-physiologiques.

Dans une première forme de réalisation de la présente invention, le piston spécifié plus haut présente une forme allongée et comporte un alésage axial dans lequel peut coulisser la tige solidaire de ce piston, l'ensemble comprenant des moyens de blocage pour fixer la position axiale relative de ce dernier et de la tige correspondante.

De préférence, ces moyens de blocage comprennent une vis qui peut être vissée dans un taraudage radial du piston pour s'appuyer sur la tige qu'il renferme.

Si cette tige est lisse, il est possible de la bloquer dans n'importe quelle position dans le piston, mais sa surface peut également présenter des évidements ou des rainures annulaires axialement espacées, auquel cas le blocage ne peut avoir lieu que dans un petit nombre de positions, égal à celui des évidements ou des rainures, mais les déplacements axiaux sont alors totalement interdits, sans qu'il soit nécessaire pour autant de serrer énergiquement la vis de blocage .

En outre, l'attelle selon l'invention peut comprendre des moyens pour bloquer provisoirement la position relative de son piston et de son cylindre. Ces moyens peuvent être constitués par une vis radiale vissée dans le corps du cylindre et s'appuyant sur le piston ; lors de la mise en place, ils permettent de régler la longueur de l'ensemble de la pièce mécanique reliant les deux organes de contention sans que l'on soit gêné par des déplacements intempestifs du piston dans son cylindre.

Dans une autre forme de réalisation de la présente invention, le piston spécifié ci-avant présente la forme d'un disque, et l'une au moins des tiges reliant l'ensemble formé par le piston et le cylindre aux organes de contention est réalisée en deux parties dont la position axiale relative est réglable, ce qui permet de fixer sa longueur, et donc celle de l'ensemble, à une valeur prédéterminée

Ce réglage peut être réalisé grâce au fait que les deux parties de la tige concernée sont vissées dans un manchon commun à filetages opposés. Il suffit alors de faire tourner ce manchon dans un sens ou dans l'autre pour écarter ou rapprocher l'une de l'autre ces deux parties de tige, et, donc, pour modifier la longueur de cette dernière.

Selon une variante, les deux parties de la tige en question peuvent coulisser télescopiquement l'une dans l'autre et être bloquées axialement l'une par rapport à l'autre, par exemple au moyen de vis.

Dans ce cas, les extrémités des sections de la tige qui peuvent coulisser l'une dans l'autre et être bloquées par une vis peuvent être entourées par deux ressorts de compression qui s'appuient, d'un part, sur une butée ménagée à l'extrémité de la tige intérieure, et, d'autre part, sur des butées ménagées, respectivement, sur les tiges intérieure et extérieure.

Enfin, et de manière avantageuse, les moyens de fixation des tiges de l'attelle sur les organes de contention correspondants peuvent comprendre une rotule qui est ménagée à une extrémité de la tige correspondante, qui peut tourner dans une cuvette sphérique solidaire de l'organe de contention concerné, et qui peut y être bloquée dans une position choisie à l'avance au moyen de vis de blocage. Ceci permet de donner à la pièce mécanique qui relie les organes de contention toute orientation souhaitée par rapport à ces derniers, et ce, dans les trois plans de l'espace.

La description qui va suivre, et qui ne présente aucun caractère limitatif, permettra de bien comprendre comment la présente invention peut être mise en pratique. Elle doit être lue en regard des dessins annexés, parmi lesquels:
- Les figures 1 et 2 représentent des vues en coupe axiale du dispositif selon l'invention dans deux de ses formes de réalisition ;
- La figure 3 montre, de manière extrêmement schématique, une variante du dispositif permettant de régler la longueur de l'attelle selon l'invention ; et :
- Les figures 4 et 5 représentent deux modes d'utilisation du dispositif selon l'invention.

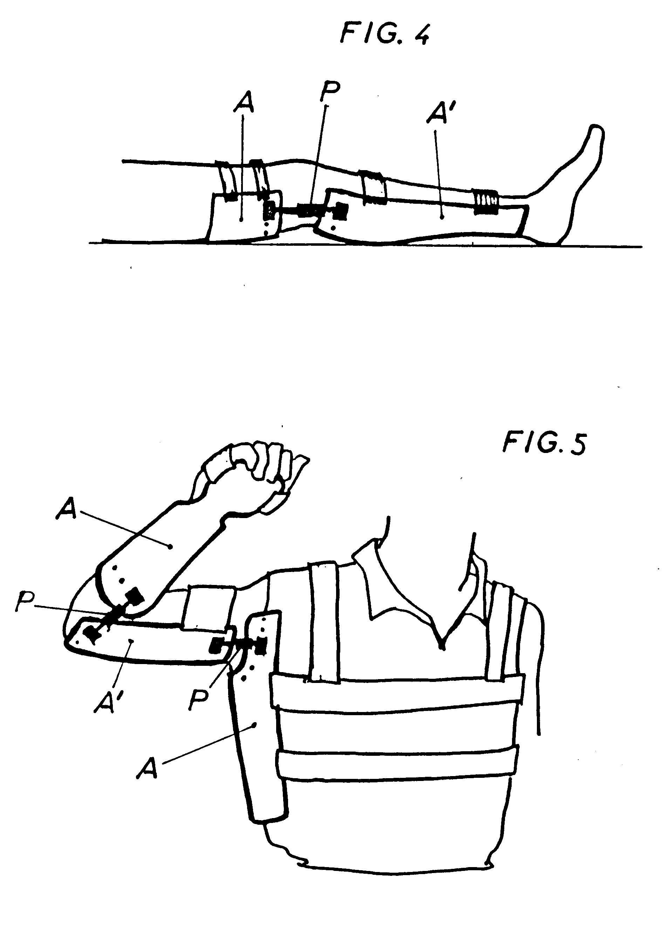

Ce dispositif comprend, pour l'essentiel, deux organes de contention A et A' (voir figures 4 et 5) reliés entre eux par une pièce mécanique P. Les organes A et A' sont réalisés en une matière thermoformable ou en tout autre matériau, et ils sont recouverts intérieurement d'un tissu mousse anti-dérapant et hypoallergique du type de celui utilisé dans les combinaisons de plongée ou d'un autre type adéquat. Ils présentent des trous en divers endroits pour permettre d'y fixer l'une des extrémités de la pièce mécanique P dans la ou les positions correspondant à l'action thérapeutique recherchée.

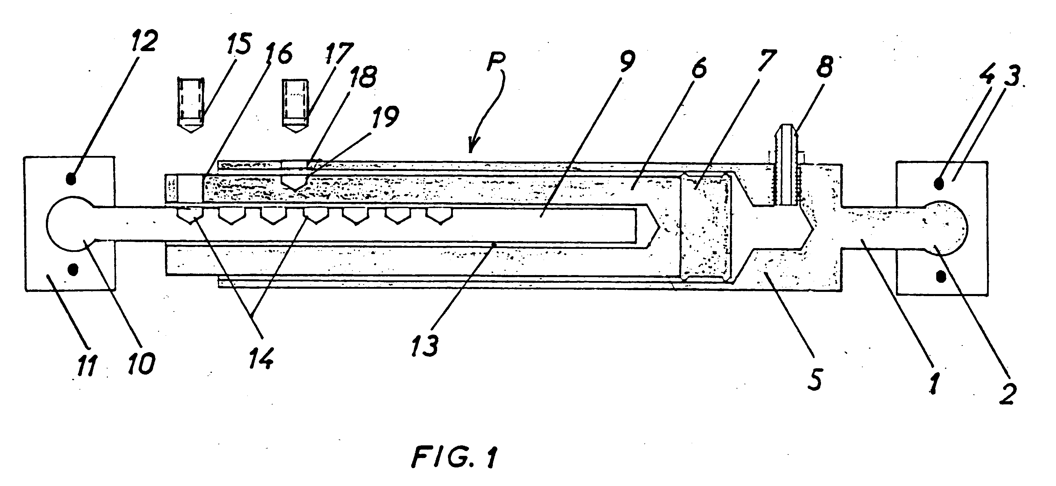

Dans une première forme de réalisation de l'invention représentée sur la figure 1, la pièce mécanique P comprend une première tige 1 dont une extrémité présente une rotule 2 qui peut tourner dans une cuvette sphérique de forme correspondante ménagée dans un bloc 3 du genre de ceux décrits dans la demande de brevet PCT/FR/OO164, la rotule 2 pouvant être bloquée en position par des vis représentées très schématiquement en 4, et le bloc 3 étant fixé sur l'un des organes de contention A ou A'. La tige 1 se prolonge axialement par un cylindre 5 dans lequel peut coulisser un piston 6 dont la tête 7 est munie d'organes d'étanchéité. Une tubulure 8 débouche dans le fond du cylindre 5 et elle peut être reliée par un conduit adéquat à une source de fluide sous pression qui peut être de l'air comprimé.

A l'autre extrémité de la pièce P, une seconde tige 9 est également munie d'une rotule 10 pouvant tourner dans un bloc 11 et y être bloquée par des vis 12, le bloc 11 étant solidaire de l'autre organe de contention A ou A'. La tige 9 peut coulisser dans un alésage 13 ménagé axialement dans le piston 6. Selon une génératrice, elle présente des évidements 14 régulièrement espacés, et une vis 15 peut être vissée dans un taraudage radial 16 ménagé à l'extrémité libre du piston 6, en dehors du cylindre 5, sa tête venant se loger dans l'un des évidements 14 choisis à volonté. Enfin, une vis 17 peut être vissée dans un taraudage 18 qui traverse le corps du cylindre 5, son extrémité se logeant dans un évidement unique 19 que comporte la surface latérale du piston 6 et qui, pour des raisons de facilité du positionnement, peut être constitué par une gorge annulaire.

Le mode de fonctionnement de ce dispositif est le suivant : on commence par régler la longueur de la pièce P en rendant le piston 6 solidaire du cylindre 5 grâce à la vis de blocage 17 qui pénètre dans l'évidement 19, en faisant coulisser la tige 9 dans le piston 6 jusqu'a atteindre la longueur voulue pour la pièce P, en rendant la tige 9 solidaire du piston 6 grâce à la vis de blocage 15 qui pénètre dans l'évidement 14 que l'on a choisi, et en désolidarisant le piston 6 du cylindre 5 par dévissage de la vis de blocage 17. Il est alors possible de mettre en route le traitement par "pompage", ainsi que cela a été dit plus haut, en introduisant périodiquement un fluide sous pression par la tubulure 8.

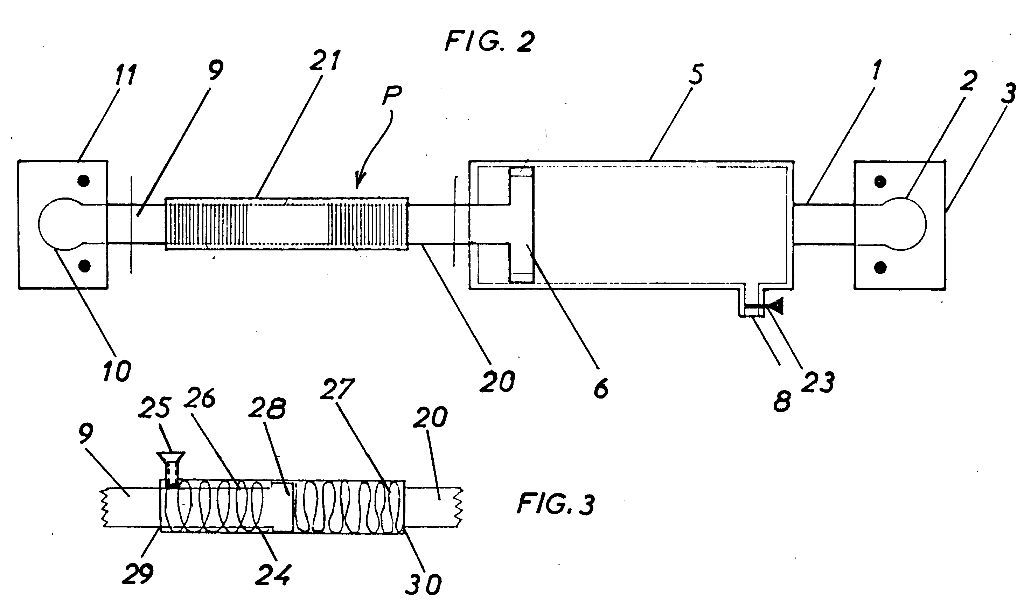

Dans une autre forme de réalisation de la pièce P, représentée sur la figure 2, on retrouve les tiges 1 et 9 prolongées, respectivement, par des rotules 2 et 10 qui sont montées dans des blocs 3 et 11 solidaires des organes de contention A et A'.

Ici, toutefois, le piston 6 présente la forme d'un disque dont la périphérie est munie des joints d'étanchéité nécessaires et qui est solidaire d'une tige axiale intermédiaire 20. Les extrémités tournées l'une vers l'autre des tiges 9 et 20 présentent des filetages de sens opposés, et elles sont vissées dans un manchon 21 fileté intérieurement.

Ainsi que cela a été dit plus haut, il est clair que si l'on fait tourner le manchon 21, les tiges 9 et 20 se rapprochent ou s'éloignent l'une de l'autre, ce qui fait varier à volonté la longueur de la pièce P. Il est d'ailleurs possible de prévoir sur le manchon 21 des dispositions rendant plus aisée cette opération, comme des cannelures, ou encore des pans ou des trous permettant l'utilisation d'une clef.

Selon une variante représentée sur la figure 3, la section de tige 20 se prolonge par un manchon 24 dans lequel peut coulisser la section de tige 9, l'extrémité de cette dernière étant entourée par une butée annulaire 28. Une vis radiale 25 traversant le corps du manchon 24 près de l'extrémité de ce dernier où passe la section de tige 9 permet de bloquer cette dernière par rapport au manchon 24, et donc, par rapport à la section de tige 20. Un ressort de compression 26 entoure la section de tige 9 entre la butée 28 et un fond 29 du manchon 24, et un autre ressort de compression 27 est logé dans le manchon 24 entre son fond opposé 30 et la même butée 28.

On comprend qu'il suffit de faire coulisser la tige intérieure 9 dans le manchon 24 de la tige 20 à l'encontre de l'un des ressorts 26 ou 27 jusqu'à ce que le système présente la longueur voulue et de bloquer l'ensemble au moyen de la vis 25 pour donner à la pièce P l'extension que l'on veut.

Quant à la phase de "pompage" sur l'articulation à traiter, elle a lieu dans le cas des dispositifs des figures 2 et 3 de la manière qui a été décrite plus haut au sujet de la forme de réalisation de la figure 1.

Les figures 4 et 5 représentent deux modes d'utilisation du dispositif selon la présente invention, respectivement dans le cas où il s'agit d'exercer un "pompage" sur un genou et dans celui où le "pompage" doit s'exercer, d'une part, sur une épaule, et, d'autre part, sur un coude. Bien entendu, les spécialistes en la matière comprendront aisément que toutes les articulations du corps peuvent être traitées de cette manière, moyennant l'utilisation d'organes de contention appropriés.

En outre, il est clair que, sans sortir du cadre de la présente invention, il serait possible d'apporter diverses modifications aux formes de réalisation qui viennent d'être décrites. C'est ainsi qu'une vanne 23 (figure 2) pourrait être montée sur la tubulure 8 d'admission de fluide sous pression. Egalement, l'axe 9 de la figure 1 pourrait être lisse, et donc ne pas comporter les évidements 14, la vis de blocage 15 étant alors une vis pointeau.

Enfin, les moyens permettant le pompage articulaire selon l'invention pourraient être, non plus pneumatiques, comme dans les exemples de réalisation préférés qui viennent d'être décrits, mais mécaniques ou électromagnétiques. De telles adaptations seraient à la portée de l'homme de l'art, et elles ne seront donc pas décrites ici de manière détaillée.

D'autre part, et bien que la description précédente ait concerné uniquement la médecine humaine, il est évident que les dispositifs selon la présente invention pourraient tout aussi bien être utilisés pour la pratique vétérinaire.

## Revendications

1. Attelle orthopédique, du genre comprenant deux organes de contention dont chacun peut être attaché à une partie du corps du patient et qui sont reliés entre eux par une pièce mécanique, pièce dont la longueur totale est réglable et qui comprend deux tiges déplaçables axialement l'une par rapport à l'autre, comprenant des moyens actifs (5, 6, 8) pour imprimer auxdites tiges (1, 9), indépendamment de la volonté du patient, des impulsions de pompage tendant à modifier leur position axiale relative, cette attelle étant caractérisé par le fait que l'extrémité libre de chacune desdites tiges (1, 9) est munie d'une rotule (2, 10) qui peut tourner dans une cuvette sphérique (3, 11) solidaire de l'organe de contention (A, A') correspondant et qui peut être bloquée dans une position choisie à l'avance au moyen de vis de blocage (4, 12).

2. Attelle selon la revendication 1, caractérisée par le fait que lesdits moyens pour imprimer auxdites tiges (1, 9) des impulsions de pompage comprennent un cylindre axial (5) dans lequel coulisse un piston (6), ce dernier et son cylindre étant chacun solidaire de l'une desdits tiges (1, 9), ainsi qu'un tubulure (8) d'admission périodique de fluide sous pression dans le fond dudit cylindre (5).

3. Attele selon la revendication 2, caractérisée par le fait que ledit piston (6) présente une forme allongée et comporte un alésage axial (13) dans lequel peut coulisser la tige (9) solidaire de ce piston (6), l'ensemble comprenant des moyens de blocage pour fixer la position axiale relative de ce dernier (6) et de la tige correspondante (9).

4. Attelle selon la revendication 3, caractérisée par le fait que lesdits moyens de blocage comprennent une vis (15) qui peut être vissée dans un taraudage radial (16) dudit piston (6) pour s'appuyer sur ladite tige (9).

5. Attelle selon la revendication 4, caractérisée par le fait que ladite vis (15) s'engage dans l'un quelconque d'une pluralité d'évidements (14) ou de rainures annulaires axialement espacées sur ladite tige (9).

6. Attelle selon l'une quelconque des revendications 2 à 5, caractérisée par le fait qu'elle comprend en outre des moyens (17, 18, 19) pour bloquer provisoirement la position dudit piston (6) dans ledit cylindre (5).

7. Attelle selon la revendication 2, caractérisée par le fait que ledit piston (6) présente la forme d'un disque et que l'une au moins desdites tiges (1, 9) est réalisée en deux parties (9, 20) vissées dans un manchon commun (21) à filetage opposés.

8. Attelle selon la revendication 2, caractérisée par le fait que ledit piston (6) présente la forme d'un disque et que l'une au moins desdites tiges (1, 9) est réalisée en deux parties (9, 20) qui peuvent coulisser télescopiquement l'une dans l'autre et être bloquées axialement l'une par rapport à l'autre.

9. Attelle selon la revendication 8, caractérisée par le fait qu'elle comprend en outre des moyens élastiques (26, 27) pour rappeler l'une vers l'autre lesdites deux parties de tige (9, 20).

## Claims

1. Orthopaedic splint of the type comprising two restraint members, each of which may be attached to a part of the patient's body and which are connected to each other by a mechanical component, the total length of which is adjustable and which comprises two rods which can be moved axially with respect to each other, comprising active means (5, 6, 8) for transmitting to the said rods (1, 9), independently of the intention of the patient, pumping impulses acting to modify their relative axial positions, this splint being characterised in that the free end of each of the said rods (1, 9) is equipped with a ball (2, 10) which can rotate in a spherical socket (3, 11) fixed to the corresponding restraint member (A, A') and which can be locked in a position chosen in advance by means of locking screws (4, 12).

2. Splint according to Claim 1, characterised in that the said means for transmitting pumping impulses to the said rods (1, 9) comprise an axial cylinder (5) in which a piston (6) slides, the latter and its cylinder each being fixed to one of the said rods (1, 9), as well as a pipe (8) for the periodic admission of fluid under pressure into the end of the said cylinder (5).

3. Splint according to Claim 2, characterised in that the said piston (6) has an elongate shape and includes an axial bore (13) in which the rod (9) fixed to this piston (6) is able to slide, the assembly comprising locking means for fixing the relative axial positions of the latter (6) and of the corresponding rod (9).

4. Splint according to Claim 3, characterised in that the said locking means comprise a screw (15) which can be screwed into a radial screw thread (16) in the said piston (6) in order to bear on the said rod (9).

5. Splint according to Claim 4, characterised in that the said screw (15) engages in any one of a plurality of recesses (14) or annular grooves axially spaced on the said rod (9).

6. Splint according to any one of Claims 2 to 5, characterised in that it also comprises means (17, 18, 19) for locking temporarily the position of the said piston (6) in the said cylinder (5).

7. Splint according to Claim 2, characterised in that the said piston (6) has the shape of a disc and that at least one of the said rods (1, 9) is made in two parts (9, 20) screwed into a common sleeve (21) with opposite threads.

8. Splint according to Claim 2, characterised in that the said piston (6) has the shape of a disc and that at least one of the said rods (1, 9) is made in two parts (9, 20) which are able to slide telescopically inside each other and be locked axially with respect to each other.

9. Splint according to Claim 8, characterised in that it also has elastic means (26, 27) for moving the said two rod parts (9, 20) back towards each other.

## Patentansprüche

1. Orthopädische Schiene umfassend zwei Halteorgane, von denen jedes an einem Körperteil des Patienten anbringbar ist und die untereinander durch ein Maschinenteil verbindbar sind, wobei das Maschinenteil, dessen Länge einstellbar ist und welches zwei axial gegeneinander verschiebbare Spindeln umfaßt, aktive Mittel (5, 6, 8) umfaßt, um auf die Spindeln (1, 9) unabhängig vom Willen des Patienten, Pumpimpulse aufzuprägen, die dazu neigen ihre relative axiale Lage zu verändern, wobei diese Schiene dadurch gekennzeichnet ist, daß das freie Ende einer jeden dieser Spindeln (1, 9) mit einem Kugelgelenk (2, 10) verbunden ist, das in einer sphärischen Schale (3, 11) drehbar gelagert ist, die fest mit dem entsprechenden Halteorgan (A, A') verbunden ist und die in einer ausgewählten Position im voraus mit Hilfe einer Blockierschraube (4, 12) blockierbar ist.

2. Schiene nach Anspruch 1, dadurch gekennzeichnet, daß die Mittel zum Aufprägen von Pumpimpulsen auf die Spindeln (1, 9) einen axialen Zylinder (5) aufweisen, in welchem ein Kolben (6) verschiebbar geführt ist, wobei der letztere und sein Zylinder fest mit jeweils einer dieser Spindeln (1, 9) verbunden sind und daß ein Rohr (8) zur periodischen Zuführung eines Fluids unter Druck im Boden des Zylinders (5) vorgesehen ist.

3. Schiene nach Anspruch 2, dadurch gekennzeichnet, daß der Kolben (6) eine langgestreckte Form und eine axiale Bohrung (13) aufweist, in welcher die Spindel (9), die mit dem Kolben (6) verbunden ist, verschiebbar ist, wobei diese Anordnung Blockiermittel zur Festlegung der relativen axialen Position des Kolbens (6) und der entsprechenden Spindel (9) aufweist.

4. Schiene nach Anspruch 3, dadurch gekennzeichnet, daß die Blockiermittel eine Schraube (15) umfassen, die in ein radiales Innengewinde (16) des Kolbens (6) zur Abstützung an der Spindel (9) einschraubbar ist.

5. Schiene nach Anspruch 4, dadurch gekennzeichnet, daß diese Schraube (15) in eine von mehreren Ausnehmungen (14) oder ringförmigen Nuten, die mit axialem Abstand auf der Spindel (9) angeordnet sind, eingreift.

6. Schiene nach einem der Ansprüche 2 bis 5, dadurch gekennzeichnet, daß sie außerdem Mittel (17, 18, 19) für die vorläufige Blockierung der Position des Kolbens (6) in dem Zylinder (5) aufweist.

7. Schiene nach Anspruch 2, dadurch gekennzeichnet, daß der Kolben (6) die Form einer Scheibe aufweist, und daß zumindest eine der Spindeln (1, 9) in zwei Teilen (9, 20) ausgeführt ist, die in eine gemeinsame Muffe (21) mit entgegengesetzten Gewinden einschraubbar sind.

8. Schiene nach Anspruch 2, dadurch gekennzeichnet, daß der Kolben (6) die Form einer Scheibe aufweist, und daß zumindest eine der Spindeln (1, 9) in zwei Teilen (9, 20) ausgeführt ist, die teleskopartig ineinander verschiebbar und in axialer Richtung gegenseitig blockierbar sind.

9. Schiene nach Anspruch 8, dadurch gekennzeichnet, daß sie außerdem elastische Mittel (26, 27) aufweist, um die beiden Teile der Spindel (9, 20) in bezug aufeinander wieder zurückzuführen.
